# EUROPEAN PATENT APPLICATION

(11) **EP 2 221 009 A1**
(43) Date of publication of application: **25.08.2010**
(21) Application number: 10250266.3
(22) Date of filing: 16.02.2010
(51) Int. Cl.: A61B 17/06, A61B 17/122, A61B 19/00

(54) **Suture management system**

(30) Priority: 18.02.2009 US 153461 P; 14.12.2009 US 636815
(71) Applicant: Tyco Healthcare Group LP, North Haven, CT 06473 (US)
(72) Inventor: Fowler, David, Cheshire, CT 06410 (US)
(74) Representative: Soames, Candida Jane

(57) **Abstract**

A system for managing one or more sutures extending from a surgical portal apparatus during a surgical procedures that incorporates the use of at least one suture includes a surgical portal member configured for insertion into a patient and defining a longitudinal passage for the receipt of the at least one suture and at least a first and a second suture clip. Each suture clip includes a base portion operably connected to a retaining portion by a hinge portion. The base portion and the retaining portion are configured for securing at least one end of a suture therebetween. The at least first and second suture clip each includes at least one visible identifying marking.

## Description

### CROSS REFERENCE TO RELATED APPLICATION

The present application claims the benefit of and priority to U.S. Provisional Application Serial No. 61/153,461 filed on February 18, 2009, the entire contents of which are incorporated herein by reference.

### BACKGROUND

### Technical Field

The present disclosure relates to trocars and other surgical portal apparatus, and more particularly, relates to a suture management system including numbered clips for use with surgical portal apparatus.

### Background of Related Art

Trocars and other surgical portal apparatus are known, as are myriad procedures that may be performed using such assemblies. Many of the minimally invasive procedures performed through access assemblies necessitate or are simplified by the use of one or more sutures passing through the surgical portal apparatus. For example, in certain procedures, such as arthroscopic procedures, it is sometimes necessary to secure soft tissue to a selected bone surface either directly or indirectly via an implant typically called an anchor. Sutures extending into a body cavity through a surgical portal apparatus may be used to, for example, temporarily retain tissue, manipulate tissue, anchor tissue or operate peripheral devices. In an attempt to reduce the number of incision sites required to complete a given surgical procedure, a single surgical portal apparatus may be used to pass one or more sutures into a body cavity, in addition to providing access for one or more devices. A single anchor device may have numerous suture ends extending through the surgical portal apparatus. These sutures may become tangled as each is manipulated or as one or more instruments are inserted and withdrawn from the assembly. Also, a surgeon may confuse the suture ends during the course of a surgery. Tangling or confusion of the suture ends may unnecessarily complicate the procedure and increase time necessary to complete the procedure.

Therefore, it would be beneficial to have a suture management system for use with a surgical portal apparatus for managing sutures during a surgical procedure such as a laparoscopic or orthopedic procedure.

### SUMMARY

A system for managing one or more sutures extending from a surgical portal apparatus during a surgical procedures that incorporates the use of at least one suture is provided. The system includes a surgical portal member configured for insertion into a patient and defining a longitudinal passage for the receipt of the at least one suture and at least a first and a second suture clip. Each suture clip includes a base portion operably connected to a retaining portion by a hinge portion. The base portion and the retaining portion are configured for securing at least one end of a suture therebetween. The at least first and second suture clip each includes at least one visible identifying marking.

The first and second suture clips may include substantially similar or substantially different visible identifying markings. The visible identifying markings on the at least first and second suture clips may be sequenced numerically and/or alphabetically. The identifying markings may be formed on the base portion and/or the retainer portion. The at least first and second suture clips may be color coded. The first and second suture clips may be selectively removable from the at least one suture. The first and second suture clips may be received on the same or different sutures.

A method of managing one or more sutures extending from a surgical portal apparatus during a surgical procedures that incorporates the use of at least one suture is provided. The method includes the steps of:
inserting a surgical portal member into a patient;
extending at least two sutures through the portal member;
attaching first and second suture clips to respective first and second sutures extending through the portal member device, the first suture clip having an identifying marker and the second suture clip having an identifying marker different from the first identifying marker; and
performing a surgical procedure with the first and second sutures as coordinated with the first and second suture clips.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
**FIG. 1** is a side view of a surgical portal apparatus and a suture management system including a pair of suture clips according to an embodiment of the present disclosure;
**FIG. 2** is a perspective view of the suture clip of **FIG. 1**, in a first or open position;
**FIG. 3** is a perspective view of the suture clip of **FIGS. 1** and **2**, in closed position;
**FIG. 4** is a perspective view of the bottom of the suture clip of **FIGS. 1-3****;**
**FIG. 5** is a cross-sectional side view of the suture clip of **FIGS. 1-4****;**
**FIG. 6** is a side view of a suture clip according to another embodiment of the present disclosure; and
**FIG. 7** is a perspective view of a clip applier for applying the clips of **FIGS. 1-6****.**
**FIG. 8** illustrates a flowchart providing the steps of a surgical procedure method, according to an embodiment of the present invention.

### DETAILED DESCRIPTION

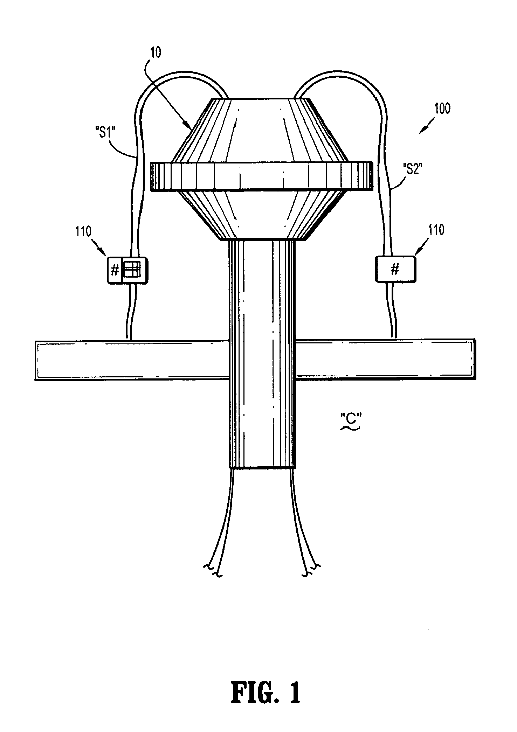

Referring now to the drawings wherein like reference numerals illustrate similar components throughout the several views, with reference initially to **FIG. 1****,** there is illustrated a surgical portal apparatus **10** and a suture management system **100** in accordance with the principles of the present disclosure. Surgical portal apparatus **10** is provided as an example only, and should not be read as limiting to the aspects of the present disclosure. Suture management system **100** may be adapted for use with any access assembly capable of receiving one or more sutures.

Still referring to **FIG. 1****,** suture management system **100** includes suture clips **110** for securing, identifying and/or manipulating respective ends of first and second sutures **"S1", "S2".** Although shown including only two suture clips, suture management system **100** may include any number of suture clips. During a procedure, a clinician may find it useful to attach more than one suture clip to a suture.

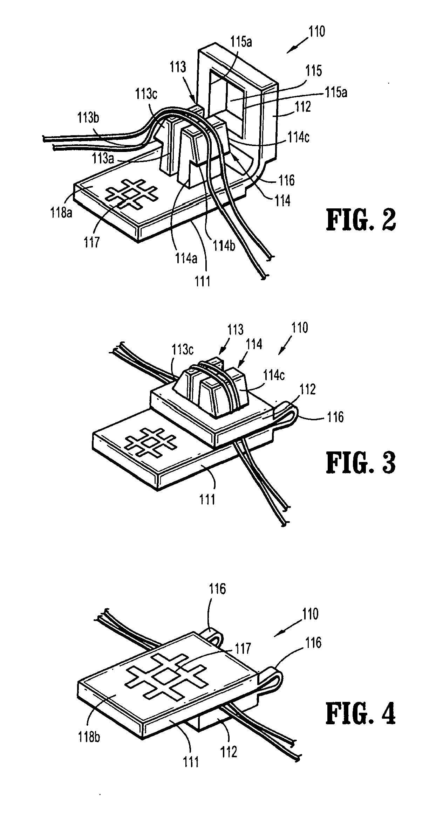

With reference now to **FIGS. 2-5****,** suture clip **110** comprises a base **111** and retainer **112** which are joined at their edges by flexible hinge **116.** Base **111** includes a pair of upstanding spaced apart prongs **113, 114.** Each prong **113, 114** includes a shaft portion **(113a, 114a,** respectively) which extends along the base, and a locking barb **(113b, 114b,** respectively) with a camming surface (**113c, 114c,** respectively). Retainer **112** includes an aperture **115** having edges 115a.

Still referring to **FIGS. 2-4****,** base **111** further includes identifying markings or labels **117** on one or both of a first and second side **118a, 118b** of base **111.** Markings **117** may include numerals, characters, symbols or any other suitable identifying character. Markings **117** may be sequentially numbered for identifying individual sutures **"S1", "S2",** or instead may include the same number, character or symbol for identifying groups of sutures. Markings **117** on suture clips **110** enable a clinician to label and/or organize sutures **"S1", "S2"** during a surgical procedure, thereby minimizing suture entanglement and confusion that might otherwise lengthen the procedure. In addition, or alternatively, clips **110** may be of different colors to distinguish clips **110,** and thus, sutures **"S1", "S2"** from one another.

To secure suture clip **110** to suture **"S1",** one or more strands of suture **"S1"** is positioned across prongs **113, 114** and retainer **112** is brought down into engagement with prongs **113, 114**. As retainer **112** is closed upon base **111,** edges **115a** of the aperture can engage surfaces **113c, 114c,** thereby forcing prongs **113, 114** to resiliently bend inward to accommodate aperture **115.** When retainer **112** and prongs **113, 114** are fully engaged, prongs **113, 114** resiliently snap outward to lock suture clip **110** closed, as shown in **FIGS. 3** and **4****.** In this manner, suture **"S1"** is held in a serpentine fashion. Although shown only receiving two strands of a single suture **"S1",** it is envisioned that suture clips **110** may be configured to hold one or more strands and that the multiple strands may be from multiple sutures.

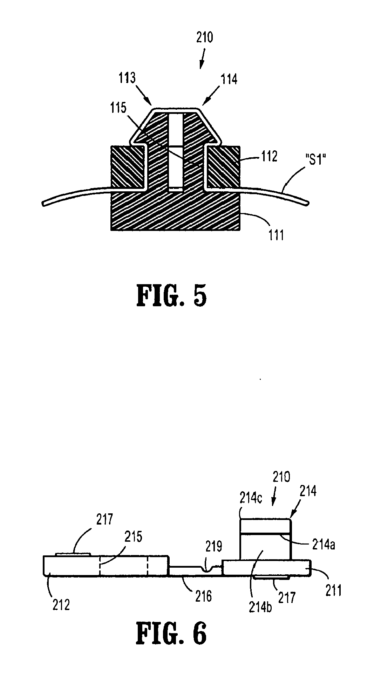

Referring now to **FIG. 6****,** an alternative embodiment of the suture clip according to the present disclosure is shown generally as suture clip **210.** Suture clip **210** includes a base portion **211** and a retainer portion **212** connected by hinge portion **216.** Retainer portion **212** includes an aperture **215** for receiving and engaging locking prongs projecting from base portion **211.** Two spaced apart prongs project perpendicularly from base **211,** prong **214** being shown in **FIG. 6****.** Both prongs include a shaft portion and a locking barb, shaft portion **214a** and locking barb **214b** being shown in **FIG. 6****.** Distal and proximal rounded edges **214c** facilitate the engagement of the prongs with aperture **215.** Hinge portion **216** includes a thin strip optionally having a laterally extending notch **219** which defines a bending region along which hinge portion **215** bends. Suture clip **210** includes at least one identifying marking **217** formed on either or both of base **211** and retainer **212.**

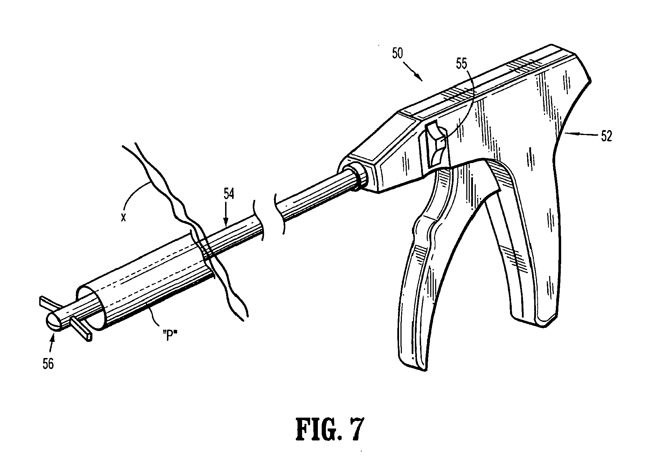

Suture clips **110, 210** may be secured to a suture manually, or may instead be applied with the assistance of a clip applier. With reference now to **FIG. 7****,** an apparatus for applying suture clips **110, 210** to one or more sutures **"S1", "S2"** is shown generally as clip applier **50.** Briefly, clip applier **50** includes a handle assembly **52,** an elongated body **54** extending from handle assembly **52** and a tool assembly **56** formed on a distal end of elongated body **54.** Handle assembly **52** includes a trigger **53** operably connected to tool assembly **56.** Handle assembly **52** further includes a rotary wheel **55** rotatably connected thereto for rotating elongated body **54.** For a more detailed discussion of clip applier **50** please refer to commonly owned U.S. Patent No. 5,645,553 to Kolesa et al., the contents of which are hereby incorporated herein in their entirety.

With reference to **FIGS. 1-5** and **7,** a procedure including the use of suture management system **100** will be described. Initially, surgical portal apparatus **10** or other surgical access assembly is used to access a body cavity **"C"** in a conventional manner as passed through a portal "p". During the course of the procedure, as one or more suture **"S1", "S2"** are used within body cavity **"C"** and the ends of sutures **"S1", "S2"** extend from surgical portal apparatus **10,** suture clips **110** are attached thereto. Suture clips **110** may be attached to suture **"S1"** manually, as described above, or may instead be attached using clip applier **50.** Once attached to suture **"S1"** markings **117** on suture clip **110** may be used to identify suture **"S1".** Additionally, suture clip **110** may facilitate manipulation of suture **"S1"** by providing a means for a clinician to grasp suture **"S1".** As sutures **"S1", "S2"** are no longer needed, clips **110** may be removed therefrom by deflecting prongs **113, 114** toward one another, thereby releasing base **111** from within aperture **115** of retainer **112.** Alternatively, sutures **"S1", "S2"** may instead be cut along the length extending between portal apparatus **10** and suture clip **110** to free sutures **"S1" "S2".**

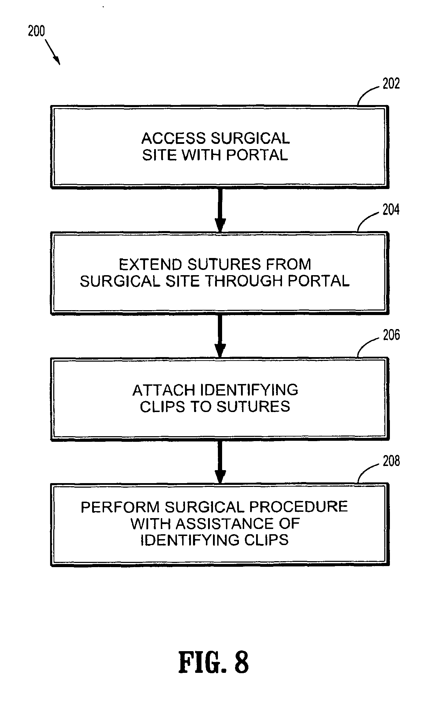

With reference to **FIG. 8****,** a surgical procedure method **200** may include the steps of inserting a surgical portal member into a patient to access an underlying body site **202;** extending at least two sutures through the portal member **204,** attaching first and second suture clips to respective first and second sutures extending through the portal member device, the first suture clip having an identifying marker and the second suture clip having an identifying marker different from the first identifying marker **206** and performing a surgical procedure with the first and second sutures as coordinated with the first and second suture clips **208.** For example, an arthroscopic procedure may be performed to attach a ligament to bone, tissue tear, repair a meniscus tear, labrum tear or the like and may incorporate the apparatus of **FIG. 7****.** The ability to identify the respective sutures associated with the procedure with the suture clips assists the clinician in properly performing the procedure, and also reduces or minimizes time spent in potentially sorting out the sutures, particularly, when a number of sutures are extending from the surgical site.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

## Claims

1. A system for managing one or more sutures extending from a surgical portal apparatus during a surgical procedures that incorporates the use of at least one suture, the system comprising:
a surgical portal member configured for insertion into a patient, the portal member defining a longitudinal passage for the receipt of the at least one suture; and
at least a first and a second suture clip, each suture clip including a base portion operably connected to a retaining portion by a hinge portion, the base portion and the retaining portion being configured for securing at least one end of a suture therebetween, wherein the at least first and second suture clip include at least one visible identifying marking.

2. The system of claim 1, wherein the first and second suture clips include substantially similar visible identifying markings.

3. The system of claim 1, wherein the first and second suture clips include different visible identifying markings.

4. The system of any preceding claim, wherein the visible identifying markings on the at least first and second suture clips are sequenced numerically.

5. The system of any of claims 1 to 3, wherein the visible identifying markings on the at least first and second suture clips are sequenced alphabetically.

6. The system of any preceding claim, wherein the at least first and second suture clips are color coded.

7. The system of any preceding claim, wherein the first and second suture clips are selectively removable from the at least one suture.

8. The system of any preceding claim, wherein the first and second suture clips are received on the same suture.

9. The system of any of claims 1 to 7, wherein the first and second suture clips are received on different sutures.

10. The system of any preceding claim, wherein the identifying markings are formed on the base portion or the retainer portion.

11. The system of any of claims 1 to 9, wherein the identifying markings are formed on both the base portion and the retainer portion.

12. A method of managing one or more sutures extending from a surgical portal apparatus during a surgical procedures that incorporates the use of at least one suture, the method comprising the steps of:
inserting a surgical portal member into a patient to access an underlying body site;
extending at least two sutures through the portal member;
attaching first and second suture clips to respective first and second sutures extending through the portal member device, the first suture clip having an identifying marker and the second suture clip having an identifying marker different from the first identifying marker; and
performing a surgical procedure with the first and second sutures as coordinated with the first and second suture clips.
